# EUROPEAN PATENT APPLICATION

(11) **EP 2 898 854 A1**
(43) Date of publication of application: **29.07.2015**
(21) Application number: 14156464.1
(22) Date of filing: 25.02.2014
(51) Int. Cl.: A61C 9/00, A61B 1/24

(54) **Intraoral cavity fixed type three-dimensional oral cavity scanner**

(30) Priority: 24.01.2014 KR 20140009055
(71) Applicant: Orapix Co., Ltd., Seoul 153-782 (KR)
(72) Inventor: Kwon, Ha Ja, 423-762 Gyeonggi-do (KR); Kang, Seok Jin, 423-762 Gyeonggi-do (KR)
(74) Representative: Hano, Christian

(57) **Abstract**

Provided is an intraoral cavity fixed type three-dimensional (3-D) oral cavity scanner (100) including: a housing (110) that can be located within an oral cavity of a patient; a photographing unit (120) that is coupled to the housing (110) and photographs part of the patient's teeth; and a fixing portion (130) that is extended from the housing (110) and that is put in gear to the patient, so that the housing is fixed within the oral cavity, to thereby relieve the discomfort of the intraoral cavity fixed type 3-D oral cavity scanner that should be directly held by user's hands and shifted from time to time.

## Description

### [Technical Field]

The present invention relates to a three-dimensional oral cavity scanner, and more particularly, to an intraoral cavity fixed type three-dimensional (3-D) oral cavity scanner.

### [Background Art]

Dental prosthetics, for example inlays, crowns, bridges, etc., are fabricated by casting a metal material or a ceramic material by using a lost wax process.

In recent years, there is a trend that a method of designing and manufacturing dental prosthetics by using a CAD/CAM system after measuring the teeth and gums by using a 3-D optical camera, replaces the lost wax method. As an example of a system measuring teeth and gums, there is the SEREC system. The SEREC system allows a 3-D oral cavity scanner using a 3-D optical camera to measure the teeth and gums to obtain the shape of the teeth in the oral cavity. A camera for executing non-contact 3-D measurements that is represented by a phase shift method or a spatial coding method is used as the 3-D optical camera. Accordingly, dental prosthetics can be manufactured by using the 3-D oral cavity scanner and CAD/CAM system more efficiently than using the lost wax method. Further, dental prosthetics having an excellent oral cavity fitness precision can be made.

An example of a conventional 3D oral cavity scanner is disclosed in Korea Patent Registration No. 10-1283635 registered on July 2, 2013.

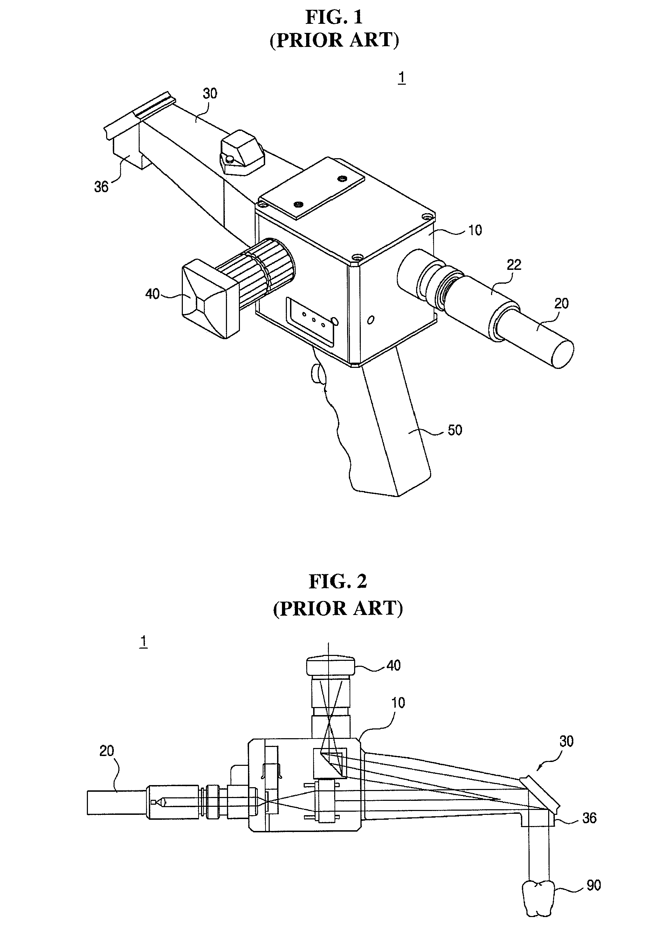

FIG. 1 is a perspective view schematically showing a conventional 3-D oral cavity scanner, and FIG. 2 is a cross-sectional view showing the conventional 3-D oral cavity scanner.

Referring to FIGS. 1 and 2, the conventional 3-D oral cavity scanner 1 includes: a case 10; a laser diode 20 that is provided at the rear side of the case 10 and irradiates laser light; a light-transmitting unit 30 that is connected to the front side of the case 10, and provided with a measuring nozzle 36 that transmits laser light generated from the laser diode 20 onto an object such as teeth and gums in the oral cavity, and receives light reflected from the object 90; and a photographing unit 40 that photographs light reflected from the object 90.

Meanwhile, in order to obtain 3-D images of an object to be measured such as the teeth and gums by using the conventional 3-D oral cavity scanner 1, a user should hold a handle portion 50 and let the light-transmitting unit 30 enter into the oral cavity, to then have the measuring nozzle 36 located at the positions of the teeth to be measured.

As a result, in order to obtain 3-D images of the teeth and gums, users undergo inconveniences of directly holding the handle portion 50 of the conventional 3-D oral cavity scanner 1 and shifting the oral cavity scanner 1 from time to time.

In addition, in the case that a patient moves, the position of the measuring nozzle 36 changes, and the quality of the acquired 3-D image is degraded. Further, in order to obtain a high-resolution 3-D image, there is the problem that an experienced operation is required in quick response to the movement of the patient.

### [Disclosure]

### [Technical Problem]

To solve the above problems or defects, it is an object of the present invention to provide an intraoral cavity fixed type three-dimensional (3-D) oral cavity scanner that can allow a user to easily acquire three-dimensional images of the teeth and gums of a patient, and further acquire high-resolution three-dimensional images of the patient's teeth and gums even when the patient moves, and that can be easily used by even unskilled persons.

The object and other objects of the present invention are not limited to the above-mentioned object, and it will be appreciated that the non-described objects and advantages of the invention may be understood by the following description, and may be understood more clearly by the description of the embodiments of the present invention. It will be also easily appreciated that the objects and advantages of the invention may be implemented by elements presented in the patent claims and combinations thereof.

### [Technical Solution]

To accomplish the above and other objects of the present invention, according to an aspect of the present invention, there is provided an intraoral cavity fixed type three-dimensional (3-D) oral cavity scanner comprising:
a housing that can be located within an oral cavity of a patient;
a photographing unit that is coupled to the housing and photographs a part of the patient's teeth; and
a fixing portion that is extended from the housing and that is put in gear to the patient, so that the housing is fixed within the oral cavity.

Preferably but not necessarily, the fixing portion is extended from one side of the housing or the other side of the housing.

Preferably but not necessarily, the entire shape of the housing and the fixing portion is a bow-shape which corresponds to the patient's teeth.

Preferably but not necessarily, the fixing portion is coupled to a cushion member that prevents damage to the patient's teeth.

Preferably but not necessarily, the photographing unit comprises: a camera module that photographs the patient's teeth and gums; and a camera module shifting unit that shifts the camera module.

Preferably but not necessarily, the camera module shifting unit makes the camera module shifted from the upper surfaces of the patient's teeth to the patient's gums.

Preferably but not necessarily, the camera module shifting unit comprises:
a fixed portion that is coupled to the housing;
a screw being rotatably coupled to the fixed portion;
a rotary motor that is coupled to the fixed portion, to thus rotate the screw; and
a camera module support plate into which the screw is rotatably fitted, and with which the camera module is coupled, wherein the camera module support plate moves in the axial direction of the screw by rotation of the screw.

Preferably but not necessarily, the screw is placed in an oblique direction with respect to the axis of the patient's teeth.

Preferably but not necessarily, a plurality of the photographing units are provided, in which some photographing units are disposed at the tongue's side of the housing and the remaining photographing units are disposed at the lip's side of the housing.

### [Advantageous Effects]

As described above, according to the present invention, the discomfort of making a user shift an intraoral cavity fixed type three-dimensional (3-D) oral cavity scanner from time to time is solved.

In addition, even when the patient's head moves, an intraoral cavity fixed type three-dimensional (3-D) oral cavity scanner can be maintained in a fixed state in an oral cavity of a patient, to thereby allow a user to acquire high-resolution three-dimensional images and further even unskilled persons to easily use the intraoral cavity fixed type 3-D oral cavity scanner.

### [Description of Drawings]

FIG. 1 is a perspective view schematically showing a conventional three-dimensional (3-D) oral cavity scanner.
FIG. 2 is a cross-sectional view showing the conventional 3-D oral cavity scanner.
FIG. 3 is a perspective view schematically showing an intraoral cavity fixed type three-dimensional (3-D) oral cavity scanner in accordance with an embodiment of the present invention.
FIG. 4 is a perspective view showing a photographing unit in accordance with an embodiment of the present invention.
FIGS. 5A through 5C are diagrams for explaining action of the photographing unit in accordance with an embodiment of the present invention.
FIG. 6 is a diagram illustrating a relationship of arrangement of the photographing unit according to an embodiment of the present invention.
FIG. 7 is a perspective view schematically showing an intraoral cavity fixed type three-dimensional (3-D) oral cavity scanner in accordance with another embodiment of the present invention.
FIGS. 8A through 8C are diagrams for explaining functions and effects of an intraoral cavity fixed type three-dimensional (3-D) oral cavity scanner in accordance with an embodiment of the present invention.

### [Best Mode]

The objects, features and advantages of the invention will become apparent through the exemplary embodiments that are illustrated in the accompanying drawings and detailed in the following description. Accordingly, the inventive technological concept can be made by those skilled in the art without departing from the spirit and scope of the invention.

Hereinafter, embodiments of the present invention will be described in detail with reference to the accompanying drawings. In this process, the size and shape of the components illustrated in the drawings can be exaggerated for convenience and clarity of explanation. Further, specifically defined terms can vary according to operator's intention or custom considering the configuration and operation of the present invention. Definitions of these terms herein need to be made based on the contents through the specification. Hereinafter, terms representing directions or positions such as up/down, left/right, and front/rear are based on the appended drawings.

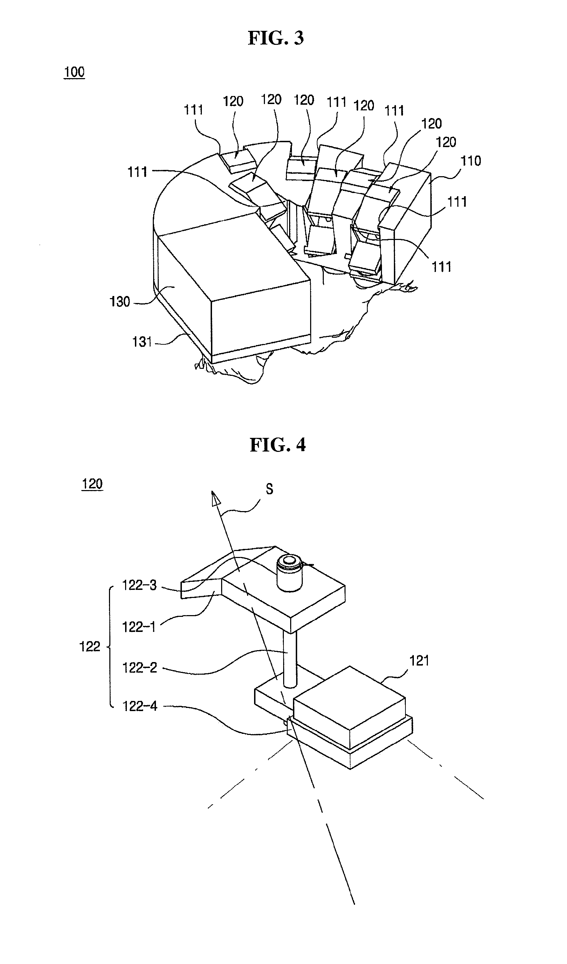

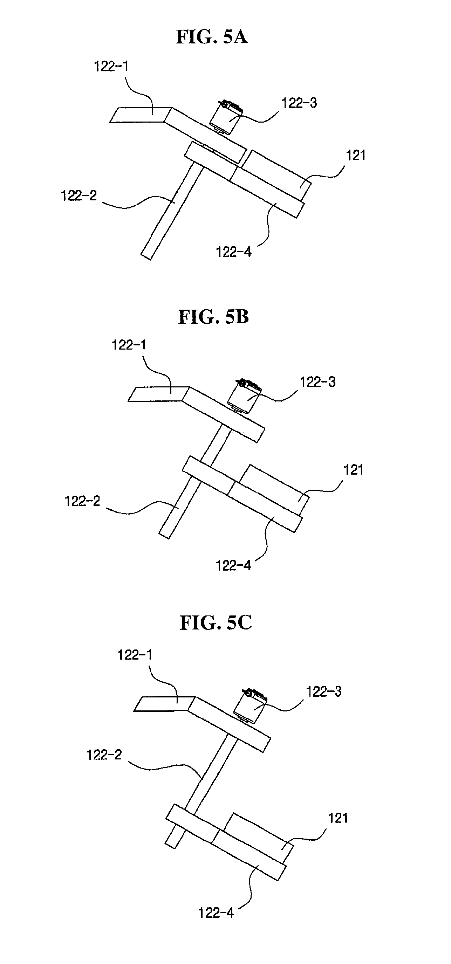

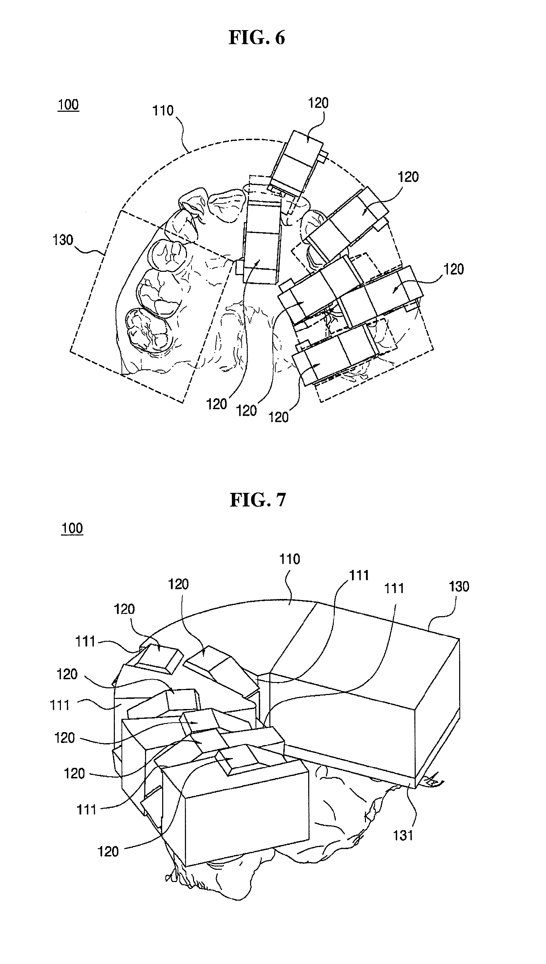

FIG. 3 is a perspective view schematically showing an intraoral cavity fixed type three-dimensional (3-D) oral cavity scanner in accordance with an embodiment of the present invention. FIG. 4 is a perspective view showing a photographing unit in accordance with an embodiment of the present invention. FIGS. 5A through 5C are diagrams for explaining action of the photographing unit in accordance with an embodiment of the present invention. FIG. 6 is a diagram illustrating a relationship of arrangement of the photographing unit according to an embodiment of the present invention. FIG. 7 is a perspective view schematically showing an intraoral cavity fixed type three-dimensional (3-D) oral cavity scanner in accordance with another embodiment of the present invention.

Referring to FIG. 3, the intraoral cavity fixed type three-dimensional oral cavity scanner 100 according to an embodiment of the present invention includes: a housing 100 that can be located within an oral cavity of a patient; a photographing unit 120 that is coupled to the housing 110 and photographs part of the patient's teeth; and a fixing portion 130 that is extended from the housing 110 and that is put in gear to the patient, so that the housing 110 is fixed within the oral cavity.

The entire shape of the housing 110 and the fixing portion 130 may be a bow shape corresponding to the shape of the patient's teeth.

A photographing unit coupling portion 111 may be formed in a part of the housing 110 in which the photographing unit 120 is coupled to the photographing unit coupling portion 111. The photographing unit coupling portion 111 can be implemented in the form of holes or grooves whose lower sides are opened so that a camera module 121 of the photographing unit 120 can look around the tooth.

A cushion member 131 can be coupled to the fixing portion 130 in which the cushion member 131 prevents damage to the patient's teeth when a patient 130 bites the fixing portion 130. A material such as rubber or sponge may be used as a material for the cushion member 131.

As shown in FIG. 4, the photographing unit 120 includes a camera module 121 that photographs part of the patient's teeth; and a camera module shifting unit 122 that shifts the camera module 121.

The camera module 121 can be implemented into a CCD (charge coupled device) camera or a CMOS (complementary metal-oxide semiconductor) camera that are generally known. Such a camera module 121 is shifted by the camera module shifting unit 122, to thus photograph the teeth at high speed.

The camera module shifting unit 122 includes: a fixed portion 122-1 that is fixed near the photographing unit coupling portion 111 of the housing 110; a screw 122-2 being rotatably coupled to the fixed portion 122-1; a rotary motor 122-3 that is coupled to the fixed portion 122-1, to thus rotate the screw 122-2; and a camera module support plate 122-4 into which the screw 122-2 is rotatably fitted, and with which the camera module 121 is coupled, wherein the camera module support plate 122-4 moves in the axial direction of the screw 122-2 by rotation of the screw 122-2.

The screw 122-1 is placed in an oblique direction with respect to the axis S of the patient's teeth, so that the camera module 121 can photograph the teeth at the tongue or lip side of the teeth.

As illustrated in FIGS. 5A to 5C, such a camera module shifting unit 122 makes the camera module 121 move up and down by the rotational force of the rotary motor 122-3.

Meanwhile, as shown in FIG. 6, a plurality of the photographing units 120 are provided to photograph half or more of the whole upper or lower teeth at a time, in which some photographing units are disposed at the tongue's side of the housing 110 and the remaining photographing units are disposed at the lip's side of the housing 110.

Therefore, the intraoral cavity fixed type three-dimensional oral cavity scanner 100 according to an embodiment of the present invention is fixed in the oral cavity to thus photograph half or more of the whole upper or lower teeth.

Here, the photographing unit 120 and the fixing portion 130 can be changed in position. That is, according to one embodiment of the present invention, the fixing unit 130 is extended from one side of the housing 110. However, as shown in FIG. 7, according to another embodiment of the present invention, the fixing portion 130 may be extended from the other side of the housing 110.

Therefore, when both the intraoral cavity fixed type three-dimensional oral cavity scanner according to one embodiment of the present invention and the intraoral cavity fixed type three-dimensional oral cavity scanner according to another embodiment of the present invention are used, the three-dimensional images of all the patient's upper and lower teeth can be acquired.

In other words, the three-dimensional images of the right half of the patient's lower teeth and the left half of the patient's upper teeth can be obtained by using the intraoral cavity fixed type three-dimensional oral cavity scanner 100 according to one embodiment of the present invention as illustrated in FIG. 3, and the three-dimensional images of the left half of the patient's lower teeth and the right half of the patient's upper teeth can be obtained by using the intraoral cavity fixed type three-dimensional oral cavity scanner 100 according to another embodiment of the present invention as illustrated in FIG. 7, to thereby acquire all the three-dimensional images of all the patient's upper and lower teeth.

Hereinbelow, the function and effect of the intraoral cavity fixed type three-dimensional oral cavity scanner according to one embodiment of the present invention will be explained with reference to the drawings.

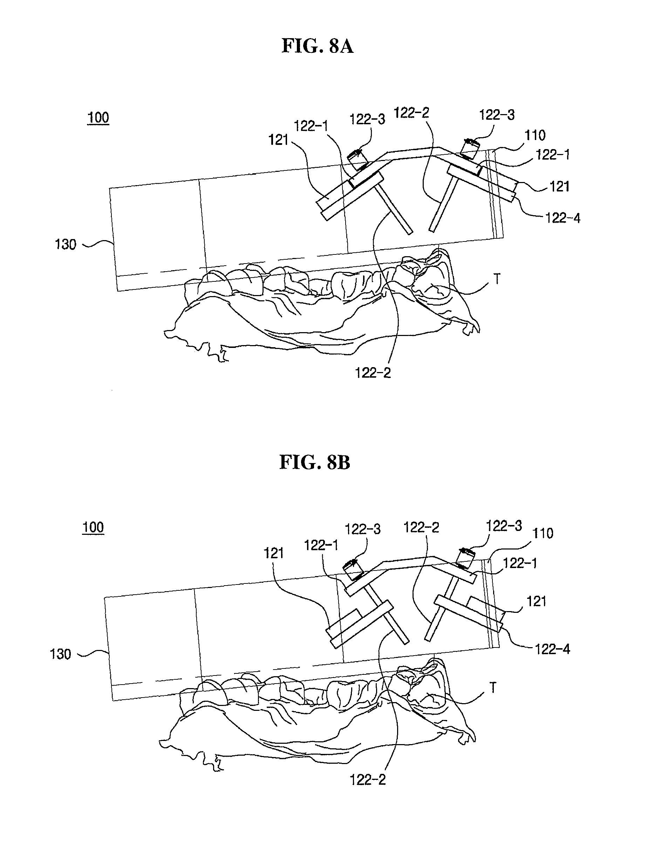

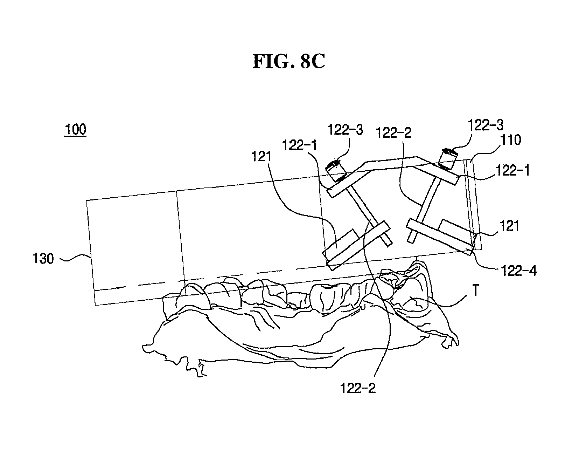

FIGS. 8A through 8C are diagrams for explaining functions and effects of an intraoral cavity fixed type three-dimensional (3-D) oral cavity scanner in accordance with an embodiment of the present invention.

First, the intraoral cavity fixed type three-dimensional oral cavity scanner 100 is arranged in the patient's oral cavity so that the lower surface of the intraoral cavity fixed type three-dimensional oral cavity scanner 100, that is, the photographing direction of the photographing unit 120 faces the teeth, and then the patient bits the intraoral cavity fixed type three-dimensional oral cavity scanner 100 so that the intraoral cavity fixed type three-dimensional oral cavity scanner 100 is fixed in the patient's oral cavity.

In this way, a control unit (not shown) controls the rotary motor 122-3 to rotate and thus controls the camera module 121 to fall down. As illustrated in FIGS. 8A through 8C, the control unit (not shown) controls the falling camera module to sequentially photograph from the upper surface of the teeth to the gums of the teeth through the side surface of the teeth, to thereby acquire the three-dimensional images of the teeth and the gums. Here, a plurality of the photographing units 120 are provided to thus obtain three-dimensional images for the plurality of the teeth.

Once a user lets the intraoral cavity fixed type three-dimensional oral cavity scanner 100 fixed in the patient's oral cavity, the intraoral cavity fixed type three-dimensional oral cavity scanner 100 does not need to move. Thus, the discomfort of making a user shift an intraoral cavity fixed type three-dimensional (3-D) oral cavity scanner from time to time is solved.

In addition, although the patient's head moves, the intraoral cavity fixed type three-dimensional oral cavity scanner 100 is maintained at a state where the intraoral cavity fixed type three-dimensional oral cavity scanner 100 is fixed in the patient's oral cavity, to thereby acquire high-resolution three-dimensional images, and even unskilled persons may easily use the intraoral cavity fixed type 3-D oral cavity scanner 100.

As described above, the present invention has been described with respect to particularly preferred embodiments. However, the present invention is not limited to the above embodiments, and it is possible for one who has an ordinary skill in the art to make various modifications and variations, without departing off the spirit of the present invention. Thus, the protective scope of the present invention is not defined within the detailed description thereof but is defined by the claims to be described later and the technical spirit of the present invention.

## Claims

1. An intraoral cavity fixed type three-dimensional (3-D) oral cavity scanner comprising:
a housing that can be located within an oral cavity of a patient;
a photographing unit that is coupled to the housing and photographs part of the patient's teeth; and
a fixing portion that is extended from the housing and that is put in gear to the patient, so that the housing is fixed within the oral cavity.

2. The intraoral cavity fixed type three-dimensional (3-D) oral cavity scanner according to claim 1, wherein the fixing portion is extended from one side of the housing or the other side of the housing.

3. The intraoral cavity fixed type three-dimensional (3-D) oral cavity scanner according to claim 1, wherein the entire shape of the housing and the fixing portion is a bow-shape which corresponds to the patient's teeth.

4. The intraoral cavity fixed type three-dimensional (3-D) oral cavity scanner according to claim 1, wherein the fixing portion is coupled to a cushion member that prevents damage to the patient's teeth.

5. The intraoral cavity fixed type three-dimensional (3-D) oral cavity scanner according to claim 1, wherein the photographing unit comprises: a camera module that photographs the patient's teeth and gums; and a camera module shifting unit that shifts the camera module.

6. The intraoral cavity fixed type three-dimensional (3-D) oral cavity scanner according to claim 5, wherein the camera module shifting unit makes the camera module shifted from the upper surfaces of the patient's teeth to the patient's gums.

7. The intraoral cavity fixed type three-dimensional (3-D) oral cavity scanner according to claim 5, wherein the camera module shifting unit comprises:
a fixed portion that is coupled to the housing;
a screw being rotatably coupled to the fixed portion;
a rotary motor that is coupled to the fixed portion, to thus rotate the screw; and
a camera module support plate into which the screw is rotatably fitted, and with which the camera module is coupled, wherein the camera module support plate moves in the axial direction of the screw by rotation of the screw.

8. The intraoral cavity fixed type three-dimensional (3-D) oral cavity scanner according to claim 7, wherein the screw is placed in an oblique direction with respect to the axis of the patient's teeth.

9. The intraoral cavity fixed type three-dimensional (3-D) oral cavity scanner according to claim 1, wherein a plurality of the photographing units are provided, in which some photographing units are disposed at the tongue's side of the housing and the remaining photographing units are disposed at the lip's side of the housing.
